# EUROPÄISCHE PATENTSCHRIFT

(11) **EP 1 140 769 B1**
(45) Veröffentlichungstag und Bekanntmachung des Hinweises auf die Patenterteilung: **14.05.2003**
(21) Anmeldenummer: 99958166.3
(22) Anmeldetag: 06.12.1999
(51) Int. Cl.: C07C 67/313, C07C 69/716, C07C 69/675, C07C 67/31, C07C 69/708, C07C 67/10, C07C 69/013

(54) **VERFAHREN ZUR HERSTELLUNG VON GLYOXYLSÄUREESTERN ODER DEREN HYDRATE**
METHOD FOR PRODUCING GLYOXYLIC ACID ESTERS OR THE HYDRATES THEREOF
PROCEDE ET DISPOSITIF POUR PREPARER DES ESTERS D'ACIDE GLYOXYLIQUE OU LEURS HYDRATES

(30) Priorität: 28.12.1998 AT 217398
(43) Veröffentlichungstag der Anmeldung: 10.10.2001
(73) Patentinhaber: DSM Fine Chemicals Austria Nfg GmbH & Co KG, 4021 Linz (AT)
(72) Erfinder: ZIMMERMANN, Curt, A-4310 Mauthausen (AT); SAJTOS, Alexander, A-4060 Leonding (AT)
(74) Vertreter: Lindinger, Ingrid
(86) Internationale Anmeldenummer: EP9909517
(87) Internationale Veröffentlichungsnummer: WO00039068

(56) Entgegenhaltungen:
- DE-A- 4 435 647
- PAVEL KRASIK: "Synthesis of Sterically Hindered Esters via Titanium Catalyzed Transesterification" TETRAHEDRON LETTERS., Bd. 39, Nr. 24, 11. Juni 1998 (1998-06-11), Seiten 4223-4226, XP002132233 ELSEVIER SCIENCE PUBLISHERS, AMSTERDAM., NL ISSN: 0040-4039 in der Anmeldung erwähnt

## Beschreibung

Glyoxylsäureester, wie etwa Ethyl-, Methyl- und Benzylglyoxylat oder L-(-)-Menthylglyoxylat sind wichtige Reagentien in der organischen Chemie, da die α-Oxo-Ester Gruppe eine sehr reaktive Gruppe darstellt, die an einer Vielzahl von Reaktionen teilnehmen kann. L-(-)-Menthylglyoxylat (MGH) ist beispielsweise ein wichtiger C₂-Baustein für die asymmetrische Synthese, für chirale Acetale, wie etwa Oxathiolane, für stereokontrollierte Additionsreaktionen an Alkenen und Nitroalkanen oder für Grignard-Reaktionen.

Die Herstellung von Glyoxylsäureestern aus den entsprechenden Maleinsäure- oder Fumarsäurediestem, mittels eines zweistufigen Ozonolyse- und Reduktionsprozesses ist bereits aus mehreren Literaturstellen bekannt.

So werden beispielsweise gemäß J. Org. Chem. 1982, 47, S 891 - 892 Ethyl-, Methyl- oder Benzylglyoxylate durch Ozonolyse der entsprechenden Maleinsäurediester in Dichlormethan, anschließender Reduktion des Ozonides mittels Dimethylsulfid und nachfolgender Destillation erhalten.

Gemäß WO 96/22960 ist ebenfalls ein zweistufiges Verfahren zur Herstellung von Menthylglyoxylat als Zwischenprodukt für Dihydroxyessigsäurementhylester beschrieben, bei welchem Dimenthylmaleat oder -fumarat in der ersten Stufe in einem Halogenkohlenwasserstoff oder Carbonsäureester, bevorzugt in Gegenwart eines niederen aliphatischen Alkohols ozonisiert wird und in der zweiten Stufe das entstandene Ozonolyseprodukt entweder mit einem Dialkylsulfid oder durch katalytische Hydrierung mit Wasserstoff zu Menthylglyoxylat reduziert wird.

Der Nachteil bei den bisher bekannten Verfahren ist jedoch, daß nach dem Ozonolyseschritt peroxidhaltige Ozonolyseprodukte vorliegen, die anschließend in einem zweiten Schritt, entweder mittels katalytischer Hydrierung oder in Anwesenheit von Dialkyl- oder Arylsulfiden, Trialkylphosphiden, zu den entsprechenden Glyoxylsäureestern reduziert werden müssen.

Zur Vermeidung dieser Probleme wurde in EP 0 884 232 A1 vorgeschlagen, z. B. MGH über Ozonolyse von Maleinsäuremonomenthylester-Natriumsalz als Edukt herzustellen. Bei diesem Verfahren entfällt zwar der bisher notwendige Reduktionsschritt, das eingesetzte Edukt ist jedoch am Markt nicht erhältlich und muß demnach in einer zusätzlichen Stufe durch Umsetzung von Maleinsäureanhydrid mit Menthol hergestellt werden.

Aus DE 44 35 647 ist weiters ein Verfahren bekannt, bei welchem eine 50 %ige Glyoxylsäure-Lösung mit einem Überschuß an Menthol mit Hilfe von Schwefelsäure und azeotroper Entfernung von Wasser verestert wird. Das Monohydrat von MGH wird durch Bildung eines Bisulfitadduktes und Phasentrennung mit anschließender Freisetzung aus dem Addukt aus dem Reaktionsgemisch isoliert.
Der Nachteil dieses Verfahrens liegt in der aufwendigen Isolierung, der notwendigen sehr schonenden Trocknung des Produktes und den erheblichen Abfallmengen.

Aus Tetrahedron Lett. 39, 4223-4226 (1998) ist die Umesterung von Glyoxylsäureethylesterdiethylacetal mit Titan-(IV)ethanolat bekannt. Bei dieser Reaktion wird jedoch erstens von einem teuren Edukt ausgegangen und zweitens ist die beschriebene Aufarbeitung des Reaktionsgemisches nach beendeter Reaktion durch Hydrolyse des Katalysators und Flashchromatographie problematisch und für den industriellen Maßstab zu teuer.

Aufgabe der vorliegenden Erfindung war es demnach ein Verfahren zur Herstellung von Glyoxylsäureestern zu finden, das oben angeführte Nachteile bisher bekannter Verfahren nicht aufweist.

Gegenstand der vorliegenden Erfindung ist daher ein Verfahren zur Herstellung von Glyoxylsäureestern, das dadurch gekennzeichnet ist, daß ein Glyoxylsäureesterhemiacetal
a) direkt mit einem Alkohol in Gegenwart eines Katalysators umgeestert wird oder
b) zuerst in das entsprechende Glyoxylsäureestervollacetal überführt und dann mit einem Alkohol in Gegenwart eines Katalysators umgeestert wird,
worauf im Anschluß an a) und b) eine Acetalspaltung zum gewünschten, freien Glyoxylsäureester oder dessen Hydrat erfolgt.

Erfindungsgemäß wird von einem Glyoxylsäureesterhemiacetal ausgegangen.
Geeignete Glyoxylsäureesterhemiacetale sind beispielweise in EP-P-0 099 981 beschrieben.
Bevorzugt sind Glyoxylsäuremethylestermethylhemiacetal (GMHA), Glyoxylsäureethylesterhemiacetale, Glyoxylsäurepropylesterhemiacetale, Glyoxylsäure-ipropylesterhemiacetale, Glyoxylsäure-t-oder-n-butylester-hemiacetale.
Besonders bevorzugt wird GMHA als Ausgangsverbindung eingesetzt.

Glyoxylsäureester bzw. deren Hydrate, die nach dem erfindungsgemäßen Verfahren erhalten werden, sind Verbindungen, deren Esterteil sich sowohl von chiralen als auch von nichtchiralen primären, sekundären oder tertiären Alkoholen ableitet.
Ester von primären Alkoholen leiten sich dabei bevorzugt von Ethanol, Butanol, Propanol und Hexanol ab. Bevorzugt werden Ester von sekundären oder tertiären Alkoholen, insbesondere von acyclischen, monocyclischen, bicyclischen Terpenalkoholen oder von acyclischen, monocyclischen, tricyclischen Sesquiterpenalkoholen, Dioder Triterpenalkoholen hergestellt, die gegebenenfalls substituiert sein können.

Besonders bevorzugte Endprodukte sind Glyoxylsäureester oder deren Hydrate, die sich von gegebenenfalls verschieden substituierten monocyclischen oder bicyclischen Terpenalkoholen, wie etwa von Mentholen, Phenylmenthol, Bomeol, Fenchol u.s.w. ableiten.

Bei dem erfindungsgemäßen Verfahren kann das Halbacetal entweder direkt (Variante a) zum gewünschten Glyoxylsäureester umgesetzt werden, oder zuerst in das entsprechende Vollacetal überführt werden (Variante b), das dann analog zu Variante a) umgeestert wird.

Die Überführung des Halbacetals in das entsprechende Vollacetal erfolgt in an sich bekannter Weise mittels eines Alkohols und saurer Katalyse.
Die Acetalisierung erfolgt dabei bevorzugt mit dem Alkohol, der bereits im Halbacetal enthalten ist. Es ist jedoch auch möglich gemischte Vollacetale herzustellen.
Geeignete Alkohole sind Methanol, Ethanol, Propanol, Butanol, Hexanol.
Bevorzugt werden die Halbacetale demnach in Glyoxylsäureesterdimethylacetale, - diethylacetale, u.s.w., überführt. Besonders bevorzugt ist das Glyoxylsäuremethylester-dimethylacetal.
Der entsprechende Alkohol wird für die Acetalisierung entweder flüssig oder dampfförmig eingesetzt. Bevorzugt erfolgt die Acetalisierung mit Alkoholdampf.
Als Katalysator eignen sich übliche Säuren, wie H₂SO₄, p-Toluolsulfonsäure, saure lonentauscher, u.s.w.
Bevorzugt wird H₂SO₄ eingesetzt.
Das sich abspaltende Wasser wird bevorzugt mit dem überhitzten Alkoholdampf ausgetragen und somit kontinuierlich aus dem Reaktionsgemisch abgezogen.
Die Umesterung der Halb- oder Vollacetale erfolgt in einem Alkohol als Reaktionsmedium. Bevorzugt werden wasserfreie Alkohole eingesetzt.

Um die oben beschriebenen Glyoxylsäureester zu erhalten wird demnach derjenige Alkohol eingesetzt, der zu dem gewünschten Esterteil im Endprodukt führt.
Dies sind demnach chirale oder nichtchirale, primäre, sekundäre oder tertiäre Alkohole, bevorzugt sekundäre oder teriäre Alkohole, insbesondere acyclische, monocyclische, bicyclische Terpenalkohole, mono- oder tricyclische, Sesquiterpenatkohole, Di- oder Triterpenalkohole.
Besonders bevorzugte Alkohole sind daher wiederum gegebenenfalls verschieden substituierte mono- oder bicyclische Terpenalkohole, wie etwa Menthole, Phenylmenthol, Borneol, Fenchol u.s.w.

Der entsprechende Alkohol kann in einer äquimolaren Menge aber auch sowohl im Überschuß als auch im Unterschuß eingesetzt werden.
So ist es bevorzugt bei billigeren Alkoholen, diese im Überschuß zum Halb- bzw. Vollacetal zuzugeben, während bei teuren Alkoholen, wie etwa Menthol u.s.w. das Acetal im Überschuß verwendet wird.

Zusätzlich zum eingesetzten Alkohol kann noch ein weiteres wasserfreies Lösungsmittel wie etwa unsubstituierte oder substituierte C₅ - C₂₀ Alkane, wie z. B. Hexan und Heptan usw., sowie Alkene, Siliziumverbindungen wie etwa Silikonöle u.s.w. oder andere unter den Reaktionsbedingungen inerte Lösungsmittel verwendet werden.

Die Umesterung findet erfindungsgemäß in Anwesenheit spezieller Katalysatoren statt. Als Katalysatoren kommen Zinn-, Titan- oder Zirkonsäureester, Lithiumverbindungen, sowie basische Katalysatoren in Frage.
Geeignete Katalysatoren sind aus der Gruppe der Zinnkatalysatoren Dialkylzinndicarboxylate mit 1- 12-C-Atomen im Alkylteil. Als Dicarboxylatteil kommen Diacetate, Dilaurate, Maleate, Diisooctoate, oder gemischte Dicarboxylate, insbesondere mit längerkettigen Fettsäureestern in Frage.

Beispiele dafür sind Dibutylzinndiacetat, Dibutylzinndilaurat, Dibutylzinndiisooctoat, Dibutylzinnmaleat, Dioctylzinndilaurat, u.s.w.
Bevorzugt wird Dibutylzinndiacetat, gemischte Dibutylzinndicarboxylate mit längerkettigen Fettsäureestem und Dioctylzinndilaurat eingesetzt.

Als Titankatalysatoren eignen sich Titan(IV)-ethanolat, -isopropanolat, -propanolat, - butanolat, -isobutanolat, u.s.w.
Bevorzugt wird Titan(IV)isopropanolat eingesetzt.
Aus der Gruppe der Zirkonkatalysatoren eignen sich Zirkonate, wie Tetrapropylzirkonat, Tetraisopropylzirkonat, Tetrabutylzirkonat, Zitronensäurediethylesterzirkonat, u.s.w.
Als Lithiumkatalysatoren können Lithiumsalze z.B. Chloride, Lithiumalkoxide oder - hydroxide aber auch organische Lithiumverbindungen wie etwa Butyllithium eingesetzt werden. Bevorzugter Lithiumkatalysator ist Butyllithium.
Insbesondere bei Variante b) können jedoch auch basische Katalysatoren wie etwa Alkali (Na,K)-verbindungen, Erdalkali(Mg) oder Aluminiumverbindungen verwendet werden. Beispiele dafür sind Hydroxide, Alkoholate oder metallorganische Verbindungen.

Bevorzugt werden Zinn-, Titan- oder Lithiumkatalysatoren verwendet.
Bei der direkten Umesterung des Halbacetals nach Variante a) werden bevorzugt Dialkyl-zinndicarboxylate als Katalysatoren zugesetzt.

Die Menge an eingesetztem Katalysator liegt bei 0,001 bis 20 mol %, bevorzugt bei 0,005 bis 5 mol% und besonders bevorzugt bei 0,02 bis 3 mol%.

Das Reaktionsgemisch wird sowohl bei Variante a), als auch bei Variante b) bevorzugt bis zum Siedepunkt des Reaktionsgemisches erhitzt, sodaß die Reaktionstemperatur in Abhänigigkeit von den Reaktanten zwischen 20°C und 200°C liegt. Die Umesterung kann weiters bei Normaldruck, aber auch bei reduziertem oder ÜberDruck von 0,001 bis 200 bar durchgeführt werden. Bevorzugt liegt der Druck zwischen 0,01 bis 10 bar, besonders bevorzugt ist Normaldruck. Der bei der Umesterung abgespaltene Alkohol wird bevorzugt kontinuierlich abdestilliert.

Wird als Alkohol ein nicht-wasserfreier Alkohol eingesetzt, so wird das Reaktionsgemisch vor Zugabe des Katalysators erhitzt, das Wasser abdestilliert und erst dann der Katalysator zugegeben.
Die Abtrennung des Katalysators nach erfolgter Umsetzung gelingt in guter Ausbeute durch Waschen mit Wasser, Hydrolyse des Katalysators und Filtraton des ausgefallenen Metalloxides oder bevorzugt durch Abdestillieren des Produktes vom Katalysator, vorzugsweise auf einem Dünnschicht- oder Kurzwegverdampfer. Es ist auch möglich, insbesondere bei der Abtrennung durch Abdestillieren des Produktes, den abgetrennten Katalysator bzw. den Destillationsrückstand einem neuen Reaktionsansatz rückzuführen.

Im Anschluß an die Umesterungsreaktion erfolgt die Acetalspaltung zum freien Glyoxylsäureester bzw. zu dessen Hydrat. Die Acetalspaltung wird mittels saurer Katalyse oder in Anwesenheit von Lanthanidenkatalysatoren durchgeführt. Als Katalysatoren für die saure Katalyse eignen sich Säuren, bei welchen die Gefahr der Hydrolyse des Esterteiles möglichst gering ist. Beispiele dafür sind H₂SO₄, p- Toluolsulfonsäure, u.s.w. und insbesonders für Variante b) Ameisensäure, Essigsäure u.s.w. Als Lanthaniden kommen diverse Verbindungen von Cer, Lanthan, Ytterbium, Samarium u.s.w. in Frage.
Dies sind insbesondere Chloride, Sulfate, Carboxylate.

Bei der Acetalspaltung werden die freien Aldehydgruppen des Glyoxylsäureesters unter Abspaltung des entsprechenden Alkoholes gebildet. Der Alkohol wird dabei bevorzugt kontinuierlich abdestilliert.

Als Endprodukt wird das Hydrat des gewünschten Glyoxylsäureesters bevorzugt, sodaß der freie Glyoxylsäureester gegebenenfalls noch durch Wasserzugabe in das Hydrat überführt wird.

In einer besonderen Ausführungsform wird das Glyoxylsäureestermethylhalb- oder Voll-acetal nach erfolgter Umesterung mittels Ameisensäure gespalten. Dabei wird durch die Reaktion des sich abspaltenden Methanols und der Ameisensäure Methylformiat und Wasser gebildet. Das Methylformiat wird abgetrennt, das Reaktionswasser bildet direkt das gewünschte Hydrat des Glyoxylsäureesters.

In einer besonders bevorzugten Ausführungsform wird das Acetal der Variante a) oder b) mit Ameisensäure kurzfristig, d.h. unter einer Stunde zum Siedepunkt erhitzt, das Methylformiat abgezogen und das verbleibende Reaktionsgemisch rasch abgekühlt.
Diese Verfahrensvariante ist bei der Herstellung des Menthylesters von besonderem Vorteil, da die Nebenproduktbildung, d.h. die Menthenbildung vermieden wird.
Restliche Ameisensäure wird extrahiert oder bevorzugt abdestilliert. Das verbleibende Reaktionsgemisch wird entweder direkt noch warm oder nach Erwärmen bevorzugt in Hexan gelöst.
Anschließend wird die Hexanlösung mit warmen Wasser gewaschen und das Endprodukt aus der organischen Phase zum Auskristallisieren gebracht.
Die Hexan-Mutterlauge kann für nachfolgende Isolierungen ohne Qualitätsverlust für das Endprodukt rückgeführt und wiedereingesetzt werden.
Bevorzugt werden die gewünschten Endprodukte mittels Variante b) hergestellt.

Mit dem erfindungsgemäßen Verfahren werden Umsätze bis zu 100 % erzielt, die Ausbeuten liegen bei über 95 %, während gemäß dem Stand der Technik (Tetrahedon) nur bis zu 80 % Ausbeute erreicht werden. Durch die schonenden Umesterungsbedingungen können Produktreinheiten von über 99,9 % bis zu 100 % erhalten werden.

### Beispiel 1:

### a) Herstellung Glyoxylsäuremethylesterdimethylacetal

1200 g (10 mol) Glyoxylsäuremethylestermethylhemiacetal und 40 g konz. Schwefelsäure wurden in einer Destillationsapparatur bestehend aus einen Sumpfbehälter, Rührer, Destillationskolonne (10 Böden) und Destillationskopf mit Rücklaufteiler auf 105°C erhitzt. Durch eine Spirale aus einem Edelstahlrohr, welche in einem Heizbad auf 110°C thermostatisiert wurde, wurden 300 g (9,4 mol) Methanol pro Stunde gepumpt. Der am Ausgang der Heizspirale austretende Methanoldampf wurde mit Hilfe eines Tauchrohres am Boden des Sumpfgefäßes in die Reaktionslösung eingeleitet. Der Rücklaufteiler am Kopf der Destillationskolonne wurde auf ein Verhältnis Abnahme zu Rücklauf von ca. 10:1 gestellt, wodurch sich rasch stationäre Bedingungen mit einer Kopftemperatur von ca. 70°C bei einer Sumpftemperatur von 105°C einstellten. Nach 6 h war die Umsetzung beendet, der Eintrag an Methanoldampf wurde gestoppt und die Heizung abgestellt. Danach wurde das Reaktionsgemisch mit festem Natriumhydrogencarbonat neutralisiert. Die Apparatur wurde evakuiert und das Reaktionsgemisch fraktioniert destilliert. Es wurden 1270 g (9,5 mol) Glyoxylsäuremethylesterdimethylacetal mit einem Gehalt von 99,5% (GC) erhalten. Die Ausbeute bezogen auf Glyoxylsäuremethylestermethylhemiacetal betrug somit 95%.

### b) Umesterung Glyoxylsäuremethylesterdimethylacetal zu L-Menthylglyoxylatdimethylacetal

402g (3 mol) Glyoxylsäuremethylesterdimethylacetal, 312g (2mol) L-Menthol und 1g Dibutylzinndiacetat wurden in der im Schritt a) beschriebenen Apparatur auf 105 °C erhitzt. Durch die Umesterung entstandenes Methanol wurde laufend am Kopf der Kolonne abgenommen. Nach 15 h war die Umsetzung beendet. Der Restgehalt an L-Menthol betrug < 0,1% (GC). Das Reaktionsgemisch wurde am Kurzwegverdampfer bei 10 mbar vom Katalysator befreit, wobei am Sumpf des Kurzwegverdampfers ca. 15g Katalysator-Lösung und im Destillat des Kurzwegverdampfers 635g Reaktionsgemisch anfielen. Das Reaktionsgemisch wurde nun im Vakuum fraktioniert destilliert. Es wurden 130 g (0,97 mol) Glyoxylsäuremethylesterdimethylacetal und 501 g (1,94 mol) L-Menthylglyoxylatdimethylacetal mit einem Gehalt von 99% erhalten. Die Ausbeute bezogen auf Glyoxylsäuremethylesterdimethylacetal betrug 97% der Theorie.
Die am Sumpf des Kurzwegverdampfers angefallene Katalysator-Lösung wurde in einem neuen Arbeitsgang anstatt des frischen Dibutylzinndiacetats eingesetzt. Nach Durchführung des Versuches mit 402g Glyoxylsäuremethylesterdimethylacetal und 312g L-Menthol wie oben angegeben wurden 509 g (1,95 mol) L-Menthylglyoxylatdimethylacetal mit einem Gehalt von 99% erhalten. Die Ausbeute bezogen auf Glyoxylsäuremethylesterdimethylacetal betrug daher 98% der Theorie.

### c) Acetalspaltung L-Menthylglyoxylatdimethylacetal zu L-Menthylglyoxylatmonohydrat

100g (0,39 mol) L-Menthylglyoxylatdimethylacetal und 400g Ameisensäure wurden in der im Schritt a) beschriebenen Apparatur 12 min zum Sieden erhitzt. Am Kopf der Kolonne wurde Methylformiat abgenommen, während die Ameisensäure bei einer Sumpftemperatur von ca. 100°C im Reaktionssystem gehalten wurde. Danach wurde das Reaktionsgemisch rasch auf Raumtemperatur abgekühlt, die Apparatur evakuiert und die Ameisensäure abgezogen. Der Rückstand wurde in 800g n-Hexan durch kurzes Erhitzen auf Siedetemperatur gelöst. Die Hexanlösung wurde zweimal mit je 400 ml 60°C heißem Wasser gewaschen. Danach wurde die Hexanlösung abgekühlt, wobei L-Menthylglyoxylatmonohydrat auskristallisierte. Die Kristalle wurden abfiltriert, der Filterkuchen mit 100 g kaltem Hexan gewaschen und im Vakuum bei Raumtemperatur getrocknet. Es wurden 64,6g (0,28 mol) L-Menthylglyoxylatmonohydrat mit einer Reinheit von 99,8 % (HPLC) erhalten. Der Drehwert (α_{D}²⁰ = -74°, c = 1 g/100ml, Acetonitril/Wasser 95:5) sowie die FTIR- und ¹H-NMR-Spektren entsprachen.
Die Mutterlauge und das Wasch-Hexan wurden vereinigt, auf 800 g eingeengt und anstatt des frischen n-Hexans in einem neuen Arbeitsgang eingesetzt. Nach Durchführung des Versuches mit 100 g L-Menthylglyoxylatdimethylacetal und 400 g Ameisensäure wie oben angegeben wurden 86,4g (0,38 mol) L-Menthylglyoxylatmonohydrat mit einer Reinheit von 99,8 % (HPLC) erhalten. Der Drehwert ( α_{D}²⁰ = -74°, c = 1 g/100ml, Acetonitril/Wasser 95:5) sowie die FTIR- und ¹H-NMR-Spektren entsprachen. Die Ausbeute betrug daher 97% der Theorie.

## Patentansprüche

1. Verfahren zur Herstellung von Glyoxylsäureestem, **dadurch gekennzeichnet, daß** ein Glyoxylsäureesterhemiacetal
a) direkt mit einem Alkohol in Gegenwart eines Katalysators umgeestert wird oder
b) zuerst in das entsprechende Glyoxylsäureestervollacetal überführt und dann mit einem Alkohol in Gegenwart eines Katalysators umgeestert wird,
worauf im Anschluß an a) und b) eine Acetalspaltung zum gewünschten, freien Glyoxylsäureester oder dessen Hydrat erfolgt.

2. Verfahren nach Anspruch 1, **dadurch gekennzeichnet, daß** als Glyoxylsäureesterhemiacetale Glyoxylsäuremethyl, ethyl-, n- oder i-propyl- oder t- oder -nbutylesterhemiacetale eingesetzt werden.

3. Verfahren nach Anspruch 1, **dadurch gekennzeichnet, daß** die Überführung in das Vollacetal mittels eines flüssigen oder dampfförmigen Alkohols aus der Gruppe Methanol, Ethanol, Propanol, Butanol, Hexanol, in Gegenwart einer Säure als Katalysator erfolgt.

4. Verfahren nach Anspruch 1, **dadurch gekennzeichnet, daß** die Umesterung mittels eines chiralen oder nichtchiralen, primären, sekundären oder tertiären Alkohols erfolgt.

5. Verfahren nach Anspruch 4, **dadurch gekennzeichnet, daß** als Alkohol ein acyclischer, monocyclischer, bicyclischer Terpenalkohol, ein acyclischer, mono- oder tricyclischer Sesquiterpenalkohol, Di- oder Triterpenalkohol eingesetzt wird.

6. Verfahren nach Anspruch 1, **dadurch gekennzeichnet, daß** als Katalysator ein Zinn-, Titan- oder Zirkonsäureester, eine Lithiumverbindung oder als basischer Katalysator eine Alkali-, Erdalkali- oder Aluminiumverbindung eingesetzt wird.

7. Verfahren nach Anspruch 6, **dadurch gekennzeichnet, daß** als Katalysator Dialkylzinndicarboxylate mit 1 - 12 C-Atomen im Alkylteil, Titan (IV)-ethanolat, -ioder n-propanolat, -n oder i-butanolat oder Butyllithium eingesetzt werden.

8. Verfahren nach Anspruch 1, **dadurch gekennzeichnet, daß** die Acetalspaltung mittels saurer Katalyse in Anwesenheit von H₂SO₄, p-Toluolsulfonsäure, Ameisensäure oder Essigsäure oder in Anwesenheit eines Lanthanidenkatalysators durchgeführt wird.

9. Verfahren nach Anspruch 8, **dadurch gekennzeichnet, daß** die Acetalspaltung durch kurzfristiges Erhitzen des Acetals bis zu 1 Stunde bis zum Siedepunkt mit Ameisensäure, Entfernen des gebildeten Formiates und raschem Abkühlen erfolgt, worauf das Produkt aus einem Verdün-nungsmittel, gegebenenfalls nach vorheriger Extraktion von Verunreinigungen mit Wasser, auskristallisiert und isoliert wird.

## Claims

1. Process for preparing glyoxylic esters, **characterized in that** it comprises
a) transesterifying a glyoxylic ester hemiacetal directly with an alcohol in the presence of a catalyst, or
b) first converting a glyoxylic ester hemiacetal into the corresponding glyoxylic ester acetal and then transesterifying it with an alcohol in the presence of a catalyst,
whereupon, following a) and b), the acetal is cleaved to give the desired free glyoxylic ester or its hydrate.

2. Process according to Claim 1, **characterized in that** the glyoxylic acid ester hemiacetals used are glyoxylic acid methyl ester, ethyl ester, n-propyl ester, isopropyl ester, or t- or n-butyl ester hemiacetals.

3. Process according to Claim 1, **characterized in that** the conversion to the complete acetal is performed using a liquid or vaporous alcohol selected from the group consisting of methanol, ethanol, propanol, butanol and hexanol in the presence of an acid as catalyst.

4. Process according to Claim 1, **characterized in that** the transesterification is performed using a chiral or nonchiral, primary, secondary or tertiary alcohol.

5. Process according to Claim 4, **characterized in that** the alcohol used is an acyclic, monocyclic, bicyclic terpene alcohol, an acyclic, monocyclic or tricyclic sesquiterpene alcohol, di- or triterpene alcohol.

6. Process according to Claim 1, **characterized in that** the catalyst used is a stannic ester, titanic ester or zirconic ester, a lithium compound or, the basic catalyst used is an alkali metal compound, alkaline earth metal compound or aluminium compound.

7. Process according to Claim 6, **characterized in that** the catalyst used is dialkyltin dicarboxylate having 1-12 carbon atoms in the alkyl moiety, titanium(IV)ethoxide, titanium(IV) isopropoxide, titanium(IV) n-propoxide, titanium(IV) n-butoxide or titanium(IV) isobutoxide, or butyllithium.

8. Process according to Claim 1, **characterized in that** the acetal is cleaved by acid catalysis in the presence of H₂SO₄, p-toluenesulphonic acid, formic acid or acetic acid, or in the presence of a lanthanide catalyst.

9. Process according to Claim 8, **characterized in that** the acetal is cleaved by brief heating of the acetal for up to 1 hour up to boiling point with formic acid, removal of the formate formed and rapid cooling, whereupon the product is crystallized out of a diluent, if appropriate after previous extraction of impurities with water, and isolated.

## Revendications

1. Procédé pour la fabrication d'esters d'acide glyoxylique, **caractérisé en ce qu'**un hémiacétal d'ester d'acide glyoxylique
a) est transestérifié directement avec un alcool en présence d'un catalyseur ou
b) est d'abord transformé en l'acétal complet d'ester d'acide glyoxylique puis transestérifié avec un alcool en présence d'un catalyseur,
et après a) et b) un clivage de l'acétal en un ester libre d'acide glyoxylique souhaité ou en son hydrate a lieu.

2. Procédé selon la revendication 1 **caractérisé en ce qu'**on utilise comme hémiacétal d'ester d'acide glyoxylique de l'hémiacétal de méthylester d'acide glyoxylique, d'éthylester d'acide glyoxylique, de n-propylester ou de i-propylester d'acide glyoxylique, ou de t-butylester ou de n-butylester d'acide glyoxylique.

3. Procédé selon la revendication 1 **caractérisé en ce que** la transformation en acétal complet avec un alcool liquide ou sous forme de vapeur du groupe méthanol, éthanol, propanol, buranol, hexanol, a lieu en présence d'un acide comme catalyseur.

4. Procédé selon la revendication 1 **caractérisé en ce que** la transestérification a lieu avec un alcool chiral ou non chiral, primaire, secondaire ou tertiaire.

5. Procédé selon la revendication 4 **caractérisé en ce que** comme alcool on utilise un alcool terpénique acyclique, monocyclique, bicyclique, un alcool sesquiterpénique acyclique, monocyclique ou tricyclique, un alcool triterpénique ou diterpénique.

6. Procédé selon la revendication 1 **caractérisé en ce que** comme catalyseur on utilise un ester d'acide stannique, d'acide de titane ou d'acide de zirconium, un composé lithium ou comme catalyseur basique un composé alcalin, alcalino-terreux ou aluminium.

7. Procédé selon la revendication 6 **caractérisé en ce que** comme catalysateur on utilise des dicarboxylates de dialkylétain ayant 1 à 12 atomes de carbone dans la part alkyle, du titan (IV)-éthanolate, titan (IV)-i-propanolate ou titan (IV)-p-propanolate, titan (IV)-n-butanolate ou titan (IV)-i-butanolate ou du butyllithium.

8. Procédé selon la revendication 1 **caractérisé en ce que** le clivage de l'acétal est réalisé avec un catalyseur acide en présence de H₂SO₄, d'acide p-toluènesulfonique, d'acide formique ou d'acide acétique ou en présence d'un catalyseur lanthanide.

9. Procédé selon la revendication 8 **caractérisé en ce que** le clivage de l'acétal a lieu par chauffage rapide de l'acétal pendant jusqu'à 1 heure au point de fusion avec de l'acide formique, élimination du formiate formé et refroidissement rapide, le produit étant cristallisé et isolé d'un diluant, le cas échéant après extraction préalable d'impuretés avec de l'eau.
